# FASCICULE DE BREVET EUROPEEN

(11) **EP 2 196 144 B1**
(45) Date de publication et mention de la délivrance du brevet: **22.05.2019**
(21) Numéro de dépôt: 09178988.3
(22) Date de dépôt: 11.12.2009
(51) Int. Cl.: A61B 5/16, A61N 1/18

(54) **Dispositif de détermination d'un état de stress chez un sujet vivant, procédé et produit programme d'ordinateur correspondants**
Vorrichtung zur Bestimmung eines Stresszustands bei einem Lebewesen, entsprechendes Verfahren und entsprechende Software
Device for determining the state of stress of a living subject, corresponding computer program product and method

(30) Priorité: 12.12.2008 FR 0858550
(43) Date de publication de la demande: 16.06.2010
(73) Titulaire: Université de Rennes 1, 35065 Rennes (FR); C.N.R.S., 35069 Rennes Cedex (FR)
(72) Inventeur: Goujon, Jean-Marc, 22300, Lannion (FR); Billon, Michel, 22300, Lannion (FR); Le Page, Ronan, 22300, Lannion (FR); Guyader, Patrick, 22300, Lanmerin (FR)
(74) Mandataire: Vidon Brevets & Stratégie

(56) Documents cités:
- EP-A1- 1 929 950
- EP-A2- 1 518 580
- WO-A1-97/40748
- WO-A1-03/057296
- WO-A1-2006/122349
- WO-A1-2008/123903
- WO-A2-2007/107900
- DE-U1- 20 022 905
- US-A- 5 143 081
- US-A- 5 311 877
- US-A- 5 595 488
- US-A1- 2004 210 261

## Description

### 1. Domaine de l'invention

Le domaine de l'invention est celui de la surveillance, de la mesure et/ou la détection d'un état de stress chez un sujet vivant, ou d'une information représentative du stress.

Plus précisément, l'invention s'applique aux dispositifs non invasifs et ambulatoires, aptes à relever une ou plusieurs informations physiologiques, placés en contact avec le corps du sujet, et par exemple portés au poignet.

L'invention peut trouver des applications dans de nombreuses situations dans lesquelles il est utile, ou souhaitable, de connaître et/ou contrôler l'état de stress d'un sujet, ou individu. Il peut notamment équiper des personnes âgées et/ou à mobilité réduite, des travailleurs isolés, des enfants, des actifs, voire des animaux.

L'invention peut notamment être mise en oeuvre pour aider à détecter les malaises, chutes, apnées du sommeil, et autres situations où le stress, conscient ou inconscient, agit sur la physiologie d'un individu.

### 2. Art antérieur

On sait que, lorsque le stress d'un individu augmente anormalement, ce qui est par exemple le cas lorsque ce dernier est soumis à un changement de situation, il développe une réaction d'alarme, encore appelée réponse de fuite ou de combat. Celle-ci est initiée par l'émission d'hormones produites par le cerveau soumis au stress, et se traduit généralement par un ensemble de réactions physiologiques telles que :
- une augmentation de la régularité du rythme cardiaque ;
- une augmentation de la pression artérielle ;
- une variation notable des paramètres respiratoires ;
- des transferts d'énergie entraînant une sudation plus importante à l'intérieur des mains ou sous les pieds ;

De manière connue, l'état de stress d'un individu peut être déterminé à partir de questionnaires ou de prélèvements biologiques permettant de mesurer le cortisol salivaire, urinaire ou sanguin.

Sont également connus des dispositifs non invasifs qui utilisent par exemple des mesures de paramètres physiologiques, tels que le rythme cardiaque, l'impédance de la peau (mesure de la résistance électrodermale), la tension artérielle, le pouls, la saturation du sang en oxygène, le rythme respiratoire, l'arythmie respiratoire, la température cutanée, le taux de sudation ou le débit sanguin.

Ces mesures sont relevées à l'aide d'un ou plusieurs capteurs physiologiques qui peuvent être intégrés à une ceinture portée par l'individu au niveau du torse ou à un bracelet porté au poignet par exemple.

Un problème lié à ces dispositifs de mesure est que la précision des mesures peut varier fortement, par exemple en fonction des conditions de port du bracelet ou de la ceinture, et des conditions environnementales de la mesure. Ainsi, quand la température est élevée, la mesure de la résistance électrodermale peut être faussée en raison de la sudation naturelle.

Ces dispositifs présentent en outre l'inconvénient majeur de fournir des signaux de mesure qui sont souvent très bruités. En effet, les mouvements relatifs d'un bracelet de mesure classique (par exemple de type boîtier montre) par rapport au poignet du sujet génèrent un bruit, dit bruit de mouvement, sur les signaux de mesure, ce qui réduit le rapport signal sur bruit des signaux de mesure. Par ailleurs, l'éloignement des capteurs physiologiques de la zone mesurée (par exemple une artère) réduit encore le rapport signal sur bruit des signaux de mesure.

Ainsi, la qualité des signaux mesurés n'est généralement pas suffisante pour fournir une mesure optimale des paramètres physiologiques du sujet et en déduire un état de stress suffisamment précis. La mesure de ces paramètres est en effet souvent aléatoire, ou à tout le moins imprécise. Ainsi, les dispositifs de mesure du stress de l'art antérieur ne fournissent pas des mesures fiables et robustes, au moins dans certaines conditions. WO 2007/107900 A2 décrit un dispositif de détermination d'un état de stress comprenant un module de mesure d'un paramètre physiologique monté sur un bracelet. EP 1 929 950 A1 décrit un dispositif de détermination d'un état de stress comprenant un module de mesure d'un paramètre physiologique et des moyens de génération d'au moins un stimulus destiné à provoquer une réponse physiologique transitoire chez ledit sujet.

### 3. Objectifs de l'invention

L'invention, dans au moins un mode de réalisation, a notamment pour objectif de pallier ces différents inconvénients de l'état de la technique.

Plus précisément, l'un des objectifs de la présente invention, dans au moins un mode de réalisation, est de fournir une technique de détermination d'un état de stress qui soit fiable et précis.

En particulier, l'invention a pour objectif de fournir, dans au moins un mode de réalisation, une telle technique qui permette d'augmenter le rapport signal sur bruit des signaux de mesure.

Par ailleurs, un objectif complémentaire de l'invention, dans au moins un mode de réalisation, est de fournir une telle technique permettant de mettre en oeuvre un dispositif :
- qui soit relativement simple et peu coûteux à fabriquer ;
- qui présente une faible consommation énergétique, et donc une bonne autonomie ;
- qui soit peu encombrant, et ergonomique (et notamment qui n'induise pas de gêne pour le porteur).

### 4. Exposé de l'invention

Ces objectifs, ainsi que d'autres, sont atteints selon l'invention à l'aide d'un dispositif de détermination d'un état de stress chez un sujet vivant, comprenant au moins un module de mesure d'au moins un paramètre physiologique dudit sujet, des moyens de génération d'au moins un stimulus destiné à provoquer une réponse physiologique transitoire chez ledit sujet et des moyens de traitement du ou des paramètres physiologiques mesurés par lesdits moyens de mesure après une durée de temporisation prédéterminée après ledit stimulus, délivrant au moins une information représentative dudit état de stress.

Selon l'invention, le module de mesure est monté sur un bracelet, destiné à être porté à un poignet dudit sujet, et porte des moyens de traitement alimentés par au moins un capteur de mesure prévu pour venir en contact avec la peau dudit sujet, au niveau dudit poignet, et lesdits moyens de génération délivrent un premier stimulus, puis, après un laps de temps, une séquence prédéterminée de signaux de sondage, ledit laps de temps étant variable, de façon pseudo-aléatoire et/ou en fonction d'un état de stress déterminé préalablement.

L'invention propose ainsi d'utiliser un dispositif apte à venir un contact avec le corps du sujet (par exemple un bracelet de type boîtier-montre tel que décrit dans la demande de brevet français 07/00841 de la Déposante) comprenant un ou plusieurs capteurs physiologiques, dont les mesures sont analysées afin de quantifier l'état de stress d'un sujet vivant, par exemple une personne âgée ou un sportif.

Le dispositif de l'invention « injecte » un stimulus au sujet vivant, déclenchant ainsi une modification transitoire, consciente ou subliminale, des paramètres physiologiques du sujet, et met en oeuvre une durée de temporisation préalablement aux mesures d'un ou plusieurs paramètres physiologiques et à l'analyse de ces mesures.

En d'autres termes, on « sonde » dans un premier temps le sujet vivant avant de mesurer son état de stress.

Les moyens de génération délivrent un premier stimulus puis, après un laps de temps, une séquence prédéterminée de signaux de sondage, également appelés « signaux d'excitation », le laps de temps pouvant être variable de façon pseudo-aléatoire et/ou en fonction d'un état de stress déterminé préalablement.

Dans au moins un des modes de réalisation de l'invention, on avertit le sujet par un stimulus qu'une mesure de son état de stress est imminente. On vise ici à provoquer l'anticipation de la réponse physiologique du sujet puis à mesurer celle-ci. Ceci permet de fiabiliser la mesure de l'état de stress du sujet.

Le stimulus peut être un signal audio, visuel (une vidéo par exemple), mécanique (vibration,...) ou électrique bref de type impulsion, ou tout événement extérieur instantané synchronisable susceptible de modifier la réponse physiologique (un choc ou une chute par exemple).

Les paramètres physiologiques mesurés sont par exemple :
- l'onde mécanique de pression générée par l'artère radiale du poignet, de laquelle on pourra déduire les intervalles entre deux impulsions et la fréquence respiratoire/cardiaque ;
- la variation de résistance dermique (aussi appelée résistance ou résistivité électrodermale, ou impédance de peau) à partir de deux électrodes ;
- la température cutanée.

Une fois le stimulus généré, le dispositif combine les données mesurées (la température, la résistance électrodermale et le rythme cardiaque par exemple) pour en déduire un niveau de stress du porteur.

Avantageusement, la réponse temporelle des signaux physiologiques mesurés est interprétée indépendamment de leur niveau d'amplitude qui peut être extrêmement variable en fonction des conditions de port du dispositif ou des conditions extérieures (par exemple, la résistance électrodermale diminue quand la température extérieure augmente, ceci étant dû à la sudation naturelle).

Afin de fiabiliser davantage la mesure, le dispositif pourra émettre un ou plusieurs stimuli supplémentaires (aussi appelés signaux d'excitation) et analyser les mesures physiologiques en réponse à chaque stimulus. La séquence de stimuli est modulable en fonction de la réponse à chaque stimulus.

Une indication de l'état de stress peut être fournie au porteur du dispositif, sous forme audio, visuelle (indicateur du type échelle graduée, vidéo,...), tactile, olfactive (parfum),... Le porteur peut, lorsque l'état de stress est supérieur à un seuil prédéterminé qui est fonction de la situation, effectuer des exercices de relaxation afin de ramener son état de stress sous ce seuil.

Inversement, si l'état de stress, appelé dans ce cas « activation », est insuffisant en regard de l'activité envisagée, le porteur peut effectuer des activités destinées à le stimuler et le rapprocher d'un niveau de stress optimal.

Dans un mode de réalisation de l'invention, le dispositif de l'invention permet en outre de détecter un malaise et/ou une chute d'un sujet vivant, telle qu'une personne âgée.

En effet, le malaise et/ou la chute constituent un fort stimulus « naturel » générant du stress, dont les conséquences physiologiques peuvent être analysées par le dispositif de l'invention lorsqu'il est porté par le sujet.

Le dispositif peut transmettre une alarme à distance lorsque le stress mesuré dépasse un seuil prédéterminé, éventuellement adapté pour chaque personne en fonction d'une situation antérieure par exemple, ceci afin d'alerter les secours par exemple.

Afin de diminuer le nombre de fausses alarmes, le dispositif de mesure de l'invention peut mettre en oeuvre des moyens de débruitage des signaux physiologiques mesurés qui permettent l'amélioration du rapport signal sur bruit des signaux de mesure.

Dans un mode de réalisation de l'invention, le dispositif de mesure met ainsi en oeuvre deux capteurs prélevant sensiblement au même point un signal représentant le même paramètre physiologique.

Dans un autre mode de réalisation de l'invention, le dispositif est en outre apte à détecter si le dispositif est porté ou non, par exemple sur la base des mesures de la température seule ou de la résistance électrodermale seule, ou une combinaison de ces signaux.

Selon un mode de réalisation de l'invention, le dispositif comprend des moyens de mémorisation d'une grandeur représentative d'au moins un paramètre physiologique mesuré avant ledit stimulus, et des moyens de comparaison des grandeurs représentatives d'au moins un paramètre physiologique mesuré avant ledit stimulus et après ledit stimulus.

De même, ladite séquence peut être variable de façon pseudo-aléatoire ou est fonction d'un état de stress déterminé préalablement à au moins un desdits signaux de sondage de ladite séquence.

Lesdits moyens de génération peuvent notamment comprendre au moins un des moyens appartenant au groupe comprenant :
- des moyens de génération d'un stimulus et/ou d'une séquence électrique ;
- des moyens de génération d'un stimulus et/ou d'une séquence visible ;
- des moyens de génération d'un stimulus et/ou d'une séquence sonore ;
- des moyens de génération d'un stimulus et/ou d'une séquence mécanique.

Par exemple, lesdits moyens de génération d'un stimulus et/ou d'une séquence sonore délivrent au moins un bruit impulsionnel et/ou au moins un fichier audio. Un stimulus mécanique peut être une vibration, et unstimulus visible peut être une vidéo.

Selon une application particulière de l'invention, lesdits moyens de traitement comprennent des moyens de détection d'une situation d'apnée du sommeil tenant compte d'au moins une information représentative de la variabilité du rythme cardiaque dudit sujet vivant mesurée par lesdits moyens de mesure et d'au moins une grandeur représentative d'un flux d'air issue de moyens d'acquisition d'un signal représentatif d'un flux d'air.

Dans ce cas, les moyens d'acquisition peuvent comprendre au moins un capteur sonore de flux d'air placé au voisinage de l'oreille dudit sujet vivant, et des moyens de communication avec lesdits moyens d'acquisition d'un flux d'air par l'intermédiaire d'une liaison sans fil peuvent être prévus.

Le dispositif peut par ailleurs comprendre des moyens de génération d'un signal de stimulation du sujet vivant mis en oeuvre dès lors qu'une situation d'apnée du sommeil est détectée.

Dans ce cas, l'amplitude et/ou la fréquence du signal de stimulation est avantageusement variable.

Notamment, le signal de stimulation peut prendre au moins deux valeurs, dont au moins une valeur commandant une stimulation suffisante pour réveiller le sujet vivant et au moins une valeur commandant une stimulation ne réveillant pas le sujet vivant.

Ce signal de stimulation peut par exemple être un signal de stimulation électrique et/ou un signal de stimulation auditive.

Par ailleurs, un tel dispositif peut comprendre, dans cette application, des moyens de détermination de l'interruption d'une situation d'apnée du sommeil tenant compte d'au moins une information représentative de la variabilité du rythme cardiaque dudit sujet vivant, d'une grandeur représentative d'un flux d'air issue de moyens d'acquisition d'un flux d'air et d'une grandeur représentative de l'impédance de la peau.

L'invention concerne également un procédé d'aide à la détermination d'un état de stress chez un sujet vivant, mettant en oeuvre un dispositif tel que décrit précédemment, comprenant les étapes suivantes :
- génération d'au moins un stimulus destiné à provoquer une réponse physiologique transitoire chez ledit sujet ;
- mesure d'au moins un paramètre physiologique après une durée de temporisation prédéterminée après ledit stimulus, délivrant au moins une information représentative dudit état de stress ;
- analyse de ladite mesure ;
- délivrance d'au moins une information représentative dudit état de stress, en fonction de ladite analyse.

Selon l'invention, le procédé comprend en outre une étape de délivrance d'un premier stimulus, puis, après un laps de temps, d'une séquence prédéterminée de signaux de sondage, ledit laps de temps étant variable, de façon pseudo-aléatoire et/ou en fonction d'un état de stress déterminé préalablement. Un tel procédé peut comprendre en outre les étapes suivantes :
- mesure d'au moins un paramètre physiologique avant ledit stimulus ;
- comparaison des mesures effectuées avant et après ledit stimulus.

Dans une application particulière, le procédé comprend une étape de détermination d'une situation d'apnée du sommeil d'un sujet vivant tenant compte d'au moins une information représentative de la variabilité du rythme cardiaque dudit sujet vivant et d'au moins une grandeur représentative d'un flux d'air émis par ledit sujet vivant.

Dans ce cas, le procédé peut en outre comprendre une étape de délivrance d'un signal de stimulation dudit sujet vivant dès lors qu'une situation d'apnée du sommeil est détectée.

Selon d'autres applications, le procédé peut comprendre une étape de détection d'une chute et/ou d'un malaise et une étape de transmission d'une alarme à distance lorsque le stress mesuré dépasse un seuil prédéterminé.

L'invention concerne également un produit programme d'ordinateur téléchargeable depuis un réseau de communication et/ou stocké sur un support lisible par ordinateur et/ou exécutable par un microprocesseur, permettant la mise en oeuvre du procédé de détermination de l'état de stress d'un sujet vivant selon tel que décrit ci-dessus, lorsqu'il est exécuté sur un ordinateur.

### 5. Listes des figures

D'autres caractéristiques et avantages des modes de réalisation de l'invention apparaîtront à la lecture de la description suivante d'un mode de réalisation préférentiel de l'invention, donné à titre d'exemple indicatif et non limitatif (tous les modes de réalisation de l'invention ne sont pas limités aux caractéristiques et avantages de ce mode de réalisation préférentiel), et des dessins annexés, dans lesquels :
- la figure 1 représente les différentes étapes du procédé de détermination d'un état de stress selon un mode de réalisation de l'invention ;
- la figure 2 représente la structure simplifiée d'un dispositif mettant en oeuvre le procédé de la figure 1 selon un mode de réalisation de l'invention ;
- la figure 3 représente un exemple d'une séquence de signaux impulsionnels (stimuli) mis en oeuvre pour déterminer l'état de stress d'un sujet vivant selon le procédé détaillé sur la figure 1 ;
- les figures 4, 5 et 6 illustrent respectivement un signal d'excitation audio émis par le dispositif de détermination d'un état de stress de l'invention et ses effets sur le rythme cardiaque et la résistance électrodermale tels que mesurés par le dispositif ;
- les figures 7A et 7B présentent des schémas d'un dispositif de détermination d'un état de stress chez un sujet vivant selon un mode de réalisation de l'invention en perspective (figure 7A) et vu de face (figure 7B);
- la figure 8 présente une coupe transversale schématique au niveau du poignet d'un individu ;
- les figures 9A et 9B présentent une vue de dessus et une vue de côté d'un schéma du module de mesure selon un mode de réalisation de l'invention ;
- la figure 10 présente un schéma de principe du palpeur du module de mesure selon un mode de réalisation de l'invention ;
- la figure 11 présente un organigramme des étapes principales d'un algorithme de traitement de l'onde mécanique de pression générée par l'artère radiale selon un mode de réalisation de l'invention ;
- la figure 12 présente un circuit électronique de traitement de l'onde mécanique de pression selon un mode de réalisation de l'invention ;
- la figure 13 présente un organigramme des étapes principales d'un algorithme de détermination de l'impédance de peau du sujet selon un mode de réalisation de l'invention ;
- la figure 14 présente un schéma d'un circuit électronique de détermination de l'impédance de peau selon un mode de réalisation de l'invention;
- la figure 15 présente un schéma d'un circuit électronique de détermination de l'impédance de peau selon une variante de l'invention ;
- la figure 16 présente un dispositif de détection de l'apnée du sommeil chez un sujet vivant utilisant un dispositif de détermination d'un état de stress de l'invention.

### 6. Description détaillée

### 6.1- Détection du stress

Le principe général de l'invention repose sur la détermination d'un état de stress chez un sujet vivant à partir de la mesure d'au moins un paramètre physiologique du sujet. Cette mesure peut être effectuée après avoir prévenu le sujet de l'imminence de la mesure. Elle est effectuée par analyse de la réponse du sujet à un stimulus bref, ou à une séquence de stimuli.

On peut ainsi exploiter la réponse temporelle, dans diverses configurations éventuelles :
- en prévenant le sujet de l'analyse imminente (anticipation),
- en appliquant un stimulus de type impulsionnel (ou une séquence de stimuli),
- en utilisant des stimuli externes brefs non prévisibles (exemple : réveil en sursaut, bruit, chute, choc...).

Cette réponse temporelle, combinaison de réponses impulsionnelles des paramètres physiologiques individuels, constitue une signature physiologique. La signature physiologique en réponse à l'événement peut permettre notamment de déterminer :
- en cas de stimulus faible, quel est l'état de stress « établi » antérieur au stimulus (analogie à la méthode des perturbations),
- en cas de stimulus de forte intensité, de détecter et caractériser l'événement (réveil suite à apnée, chute, forte émotion, etc.), notamment en fonction du niveau de stress qu'il génère.

En d'autres termes, on alerte dans un premier temps le sujet qu'une mesure d'un état de stress va être effectuée, puis on initie un temps d'attente prédéterminé préalablement à la mesure. Une fois les paramètres physiologiques mesurés, ceux-ci sont analysés et une information représentative de l'état de stress du sujet vivant est délivrée.

Le fait d'avertir le sujet que l'on va effectuer une mesure de son état de stress permet d'exacerber le caractère stressé du sujet et permet, par conséquent, de fiabiliser la mesure de l'état de stress.

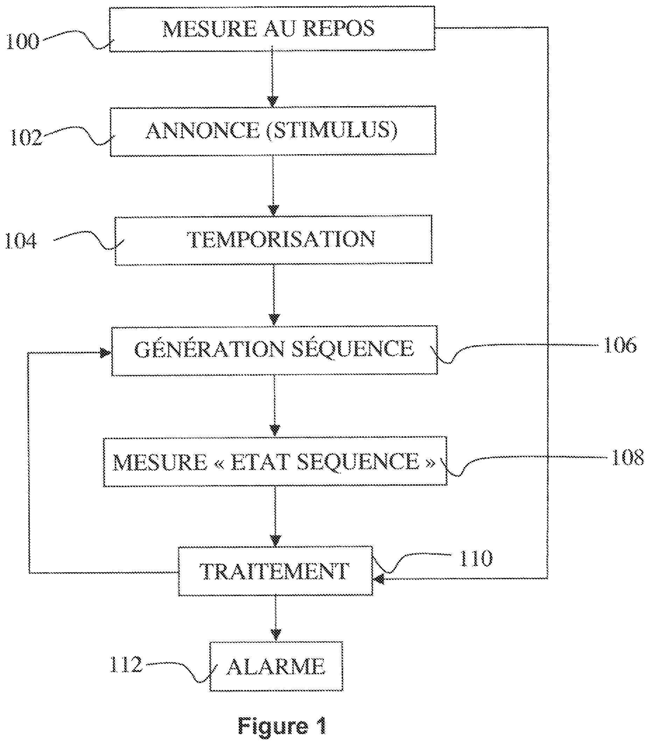

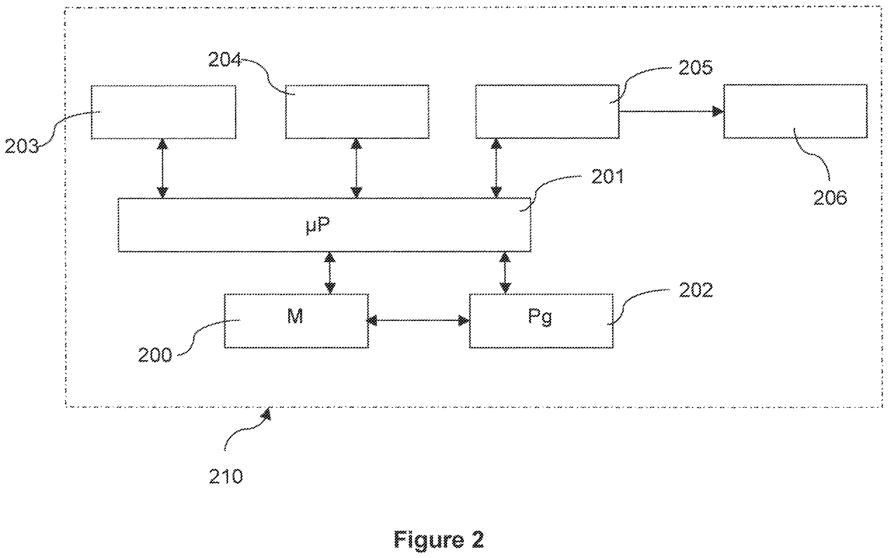

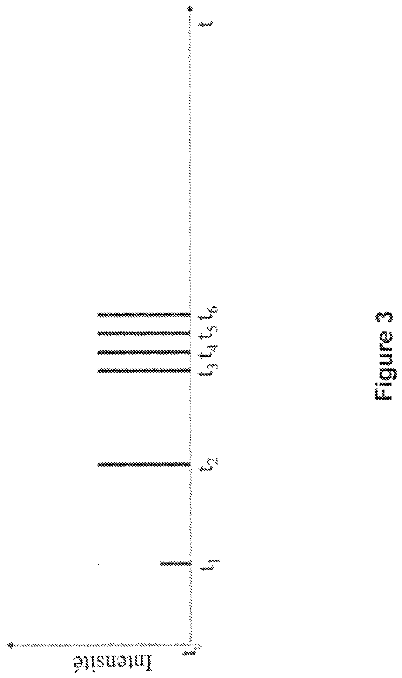

On a représenté sur la figure 1 les différentes étapes du procédé de détermination d'un état de stress d'un sujet vivant selon un mode de réalisation de l'invention. Un tel procédé est mis en oeuvre dans le dispositif de la figure 2 qui est porté par le sujet (ou porteur) et qui est apte à générer une séquence de signaux électriques impulsionnels, d'une durée inférieure à 1 minute par exemple. Une telle séquence est illustrée sur la figure 3.

On suppose ainsi qu'au temps t=0, un test de détermination de l'état de stress du sujet est déclenché automatiquement par le dispositif 210. On pourra envisager qu'un tel test soit déclenché manuellement par le sujet en appuyant sur un bouton d'activation (non représenté) du boîtier montre ou à distance. Le dispositif 210 initie alors la séquence de signaux de sondage (également appelés signaux d'excitation) impulsionnels de la figure 3.

Ainsi, aucun signal d'excitation n'est émis jusqu'au temps t=tl. Pendant l'intervalle t=0 à tl, les paramètres physiologiques (pulsation, résistance électrodermale, température, etc), autrement appelés paramètres physiologiques de repos, sont mesurés et mémorisés (étape 100) par le dispositif 210.

Au temps t=tl (égal à 5 secondes par exemple), un signal électrique de faible amplitude (ou stimulus) prévient l'utilisateur qu'une détermination de son état de stress est sur le point de commencer (étape 102).

En fonction des paramètres physiologiques mesurés à l'étape 100, de leur variabilité et de leur évolution au cours du laps de temps t=0 à tl, le dispositif 210 détermine l'instant d'apparition t2 du premier signal impulsionnel de la séquence. Le laps de temps ou temporisation t1 à t2 (étape 104) est dans ce mode de réalisation variable et vise, comme souligné précédemment, à exacerber le caractère stressé de la personne afin de fiabiliser la mesure de l'état de stress.

Une fois que le premier signal d'excitation impulsionnel de la séquence est généré à l'instant t2 (étape 106), les paramètres physiologiques mesurés (étape 108) par le dispositif 210 sont analysés et comparés (étape 110) aux paramètres physiologique de repos précédemment mesurés.

L'algorithme de traitement mis en oeuvre à l'étape 110 peut utiliser une multitude de techniques de calcul, d'association et de comparaison (logique floue, réseaux bayésiens, réseaux de neurones, analyse discriminante, ...) avec les paramètres précédemment mesurés (à l'étape 110) en tenant compte de l'historicité de ces paramètres.

L'algorithme peut mettre en oeuvre un arbre décisionnel permettant la prise de décision à partir de la classification des paramètres physiologiques selon certaines catégories prédéfinies, ou définies lors de l'étape 100.

En fonction des réponses physiologiques de l'individu à chaque signal impulsionnel de la séquence, la suite de la séquence de signaux impulsionnels est aménagée (fréquence variable).

Le deuxième signal d'excitation impulsionnel de la séquence est émis à l'instant t3 (étape 106) et de nouveau les paramètres physiologiques mesurés (étape 108) sont analysés (étape 110).

À la fin de la séquence à l'instant t6, un état de stress « final » est déterminé par le dispositif 210 (étape 110). Lorsque l'état de stress déterminé dépasse un seuil prédéterminé, une alarme sonore, visuelle ou olfactive peut être générée par dispositif 210 (étape 112) afin d'avertir le sujet qu'il est nécessaire qu'il se calme.

L'état de stress peut être représenté sur une échelle graduée du dispositif 210. Le sujet pourra, lorsqu'une alarme est émise, effectuer un exercice de respiration (type yoga) pour tenter de diminuer son état de stress.

L'alarme émise à l'étape 112 peut éventuellement être transmise à distance par des moyens de liaison sans fil du dispositif, vers un professionnel de santé par exemple.

Alternativement, le signal d'excitation peut être un signal audio comprenant des impulsions sonores brèves, de type « bips sonores », un tel signal étant délivré par une source audio (non représentée) du dispositif 210, ou un mélange de signaux électriques et audio.

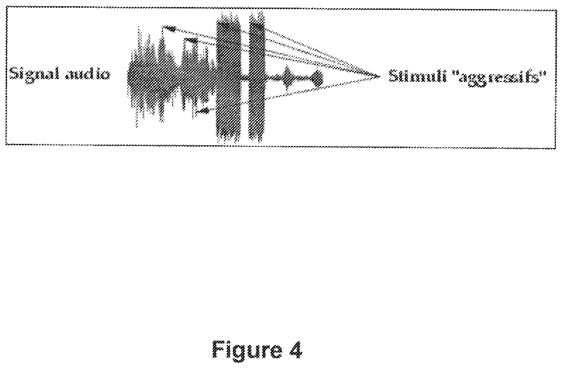

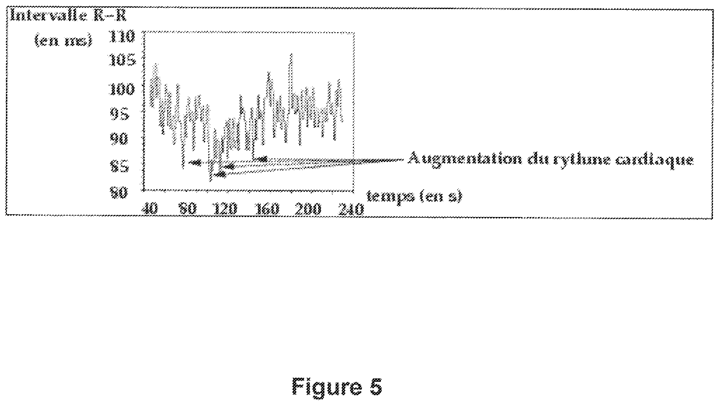

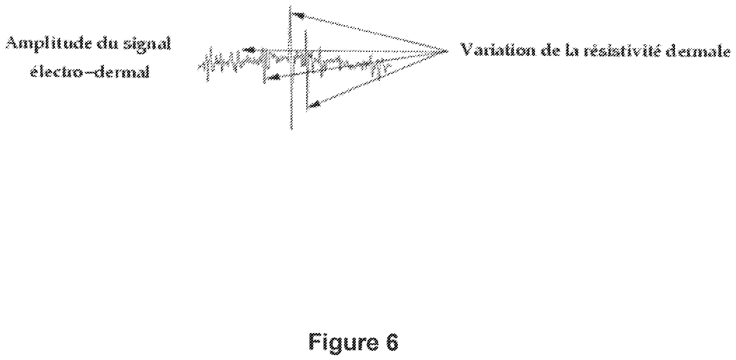

On a représenté sur la figure 4 un exemple de stimulus audio et ses effets sur le rythme cardiaque et la résistance électrodermale dont les variations sont représentées respectivement sur les figures 5 et 6. On observe ainsi qu'à chaque signal d'excitation, ou stimulus, la période du rythme cardiaque diminue et que, par conséquent, sa fréquence augmente (figure 5). Le signal électrodermal comprend des pics de température (figure 6) correspondant à chaque excitation ou stimulus.

Les différentes mesures physiologiques effectuées au cours de la séquence illustrée en figure 3 peuvent être mémorisées dans le dispositif 210 pour une utilisation future lors d'une nouvelle évaluation de l'état de stress du sujet.

La figure 2 représente la structure simplifiée d'un dispositif 210 mettant en oeuvre un tel procédé selon un mode de réalisation de l'invention. Le dispositif 210 comprend une mémoire 200 constituée d'une mémoire tampon, une unité de traitement 201 équipée par exemple d'un microprocesseur µP, et pilotée par le programme d'ordinateur 202 mettant en oeuvre le procédé de détermination d'un état de stress selon l'invention.

À l'initialisation, les instructions de code du programme d'ordinateur 202 sont par exemple chargées dans une mémoire RAM avant d'être exécutées par le processeur de l'unité de traitement 201. Le microprocesseur de l'unité de traitement 201 met en oeuvre les étapes du procédé de détermination d'un état de stress selon les instructions du programme d'ordinateur 202, pour délivrer au moins une information représentative d'un état de stress.

Pour cela, le dispositif comprend, outre la mémoire tampon 200, des moyens de génération 203 d'un stimulus destiné à alerter le sujet et des moyens d'analyse 204 du ou des paramètres physiologiques du sujet mesurés par des moyens de mesure 205. Les moyens d'analyse 204 sont aptes à délivrer au moins une information 206 représentative de l'état de stress après une durée de temporisation prédéterminée après le stimulus. Ces moyens sont pilotés par le microprocesseur de l'unité de traitement 201.

### 6.2 - Exemple de dispositif : architecture d'un bracelet de mesure

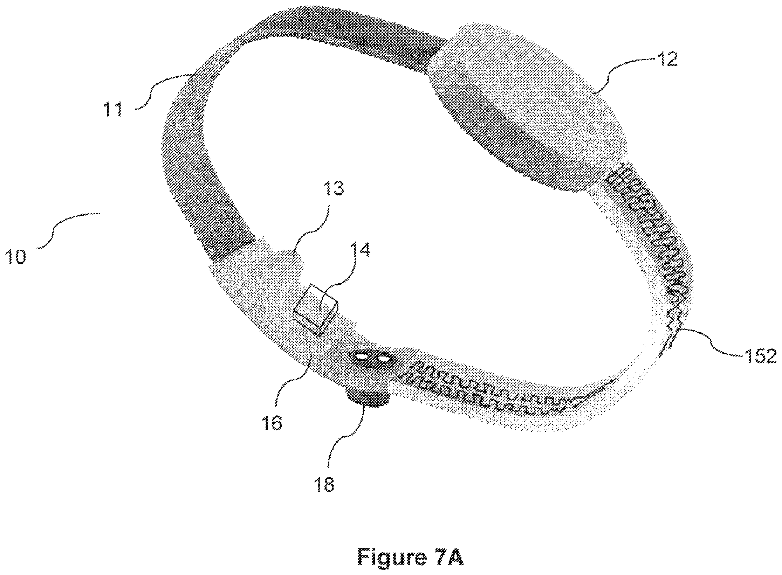

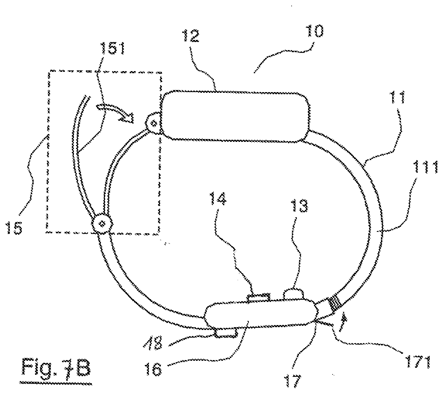

On décrit ci-après, en relation avec les figures 7A et 7B, un exemple de dispositif de détermination 10 de l'état de stress d'un sujet vivant, sous la forme d'un bracelet, selon un mode de réalisation préférentiel de l'invention en perspective (figure 7A) et vu de face (figure 7B).

Ainsi, selon le mode de réalisation préférentiel de l'invention, le dispositif de mesure 10 est un bracelet de mesure qui comprend notamment :
- un bracelet de maintien 11 qui comprend avantageusement une partie souple 111 (mais de préférence non élastique), par exemple fabriquée en élastomère 61 shores ;
- un module 16 de mesure comprenant des moyens de calage présentant au moins une protubérance (ci-après désignée par palpeur) 13 faisant saillie à l'intérieur du bracelet afin de caler le module 16 de mesure au niveau d'un poignet du sujet. Dans le palpeur 13 sont intégrés un premier ensemble de capteurs physiologiques ci-après décrits et un second ensemble 14 de capteurs physiologiques externes au palpeur, le palpeur 13 s'étendant sur une majeure partie de la largeur du module 16, sensiblement perpendiculairement à l'axe défini par le bracelet 11 ;

- un module d'excitation 18 comprenant une source électrique de stimulation à décharge piézo-électrique. Le module d'excitation 18 est dans ce mode de réalisation situé à proximité du palpeur 13 ;
- un système 12 de traitement et d'affichage d'informations obtenues notamment à partir des mesures des paramètres physiologiques ou de toute variation et/ou combinaison de ces grandeurs, le système 12 permettant en outre d'évaluer l'état de stress du porteur à partir d'au moins une de ces mesures et d'afficher une information représentative d'un état de stress. Un tel système 12 est ici intégré dans un boîtier montre comprenant une source d'énergie, une source audio et des moyens de liaison sans fil (non représentés) ;
- un système de fixation et/ou de serrage 15 par exemple à base d'une bande d'attache 151 (par exemple une bande VELCRO, marque déposée) qui permet le placement du bracelet de mesure d'une seule main sans déplacer le palpeur 13 de l'artère radiale ;
- un système de réglage 17 réversible de la longueur de la partie souple 111 entre le système de traitement et d'affichage 12 et le module de mesure 16 permettant notamment de placer au mieux le palpeur 13 et le module d'excitation 18 au niveau du poignet de l'utilisateur. Ainsi, le système de réglage 17 permet, par exemple, de faire pénétrer une partie du bracelet 11 à l'intérieur du module de mesure 16 réduisant ainsi la longueur de la partie souple 111 entre le système 12 et le module de mesure 16. Lorsque l'utilisateur considère que la position du palpeur est optimale (et donc la longueur de la partie souple 111), il peut verrouiller cette position au moyen d'une attache 171 (appartenant au système de réglage 17).

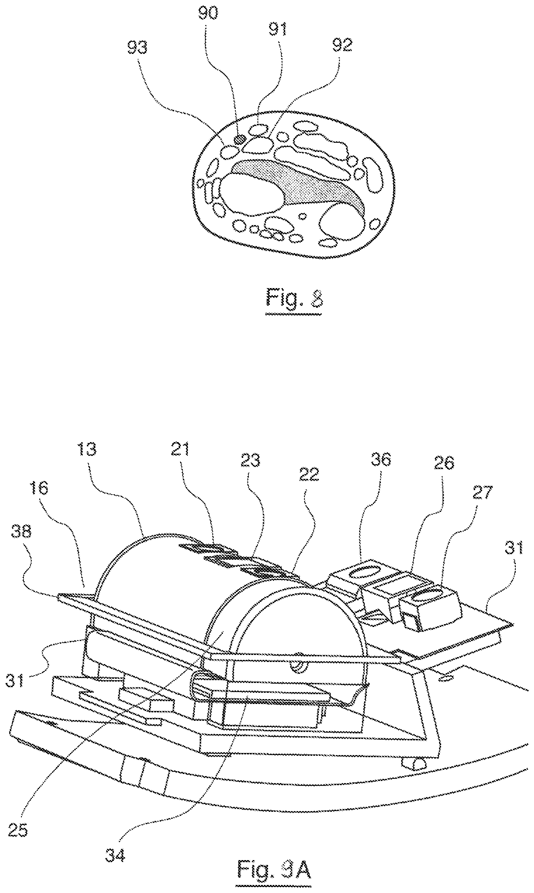

Dans ce mode de réalisation, le palpeur 13, dont la partie supérieure est par exemple de forme sensiblement demi-cylindrique, est prévue pour se positionner de manière stable dans la partie molle du poignet du sujet située à proximité de l'artère radiale 90 qui est entourée de tendons 91, 92, 93, tels qu'illustrés sur la figure 8. Ainsi, la forme de la protubérance est définie de façon à s'inscrire entre deux tendons du poignet. Par ailleurs, la forme demi-cylindrique du palpeur 13 permet également de minimiser la sensation d'inconfort d'un utilisateur lors d'un port prolongé du bracelet de mesure en épousant la partie molle du poignet.

Ainsi, le bracelet de mesure 10 permet par exemple, grâce à la protubérance, de stabiliser le module de mesure par rapport au poignet, ce qui permet de réduire le bruit sur le ou les signaux issu du ou des capteur(s) physiologique(s) dû aux mouvements relatifs du bracelet par rapport au poignet.

Il permet également d'approcher le ou les capteur(s) physiologique(s) de la protubérance (ou palpeur) au plus près de l'artère radiale du poignet du sujet ce qui permet de réduire le bruit dû à l'éloignement entre le ou les capteurs et l'artère.

Le module d'excitation 18 à décharge piézo-électrique est prévu pour se positionner dans la partie molle du poignet du sujet située à proximité de l'artère radiale 90 ou du canal ulnaire, trajets privilégiés des nerfs. Il permet l'émission d'un ou plusieurs stimuli sous la forme de signaux électriques brefs, préalablement ou ultérieurement à la mesure des données physiologiques, dans le but de déterminer l'état de stress du porteur.

Un tel bracelet de mesure 10 réduit le bruit présent sur les signaux de mesure, par un positionnement précis des capteurs, et une absence de mouvements de ceux-ci.

Les capteurs physiologiques du présent bracelet de mesure 10 peuvent par exemple délivrer au moins un signal utile représentatif d'au moins un paramètre physiologique appartenant au groupe comprenant notamment :
- l'onde de pression mécanique, avec laquelle on peut déduire le pouls, la variabilité du pouls et la fréquence respiratoire ;
- la saturation du sang en oxygène ;
- l'impédance de peau ;
- la tension artérielle ;
- le rythme respiratoire, l'arythmie respiratoire et/ou l'amplitude respiratoire ;
- la température cutanée ;
- la sudation localisée sous le bracelet de mesure au niveau du poignet.

Le fait que l'ensemble des capteurs soit intégré dans le module de mesure du bracelet de mesure permet d'obtenir une miniaturisation du système de mesure de paramètres physiologiques et de l'état de stress.

De manière optionnelle, et tel qu'illustré par la figure 7A, selon un autre mode de réalisation de l'invention, le bracelet de mesure 11 peut comprendre une bande 152 de circuit imprimé souple permettant d'assurer la transmission de signaux électriques entre les modules de mesure 16 et d'excitation 18 et le système 12 de traitement et d'affichage, par exemple pour permettre l'affichage de la valeur de l'impédance de peau de l'utilisateur ou d'une valeur représentative de son état de stress.

En outre, le module d'excitation 18 peut être activé manuellement par le porteur du bracelet en appuyant par exemple sur un bouton d'activation (non représenté) situé sur le boîtier montre du bracelet.

De façon alternative, le module d'excitation 18 peut être activé automatiquement, de manière périodique ou aléatoire, par le système de traitement et d'affichage 12.

Le module de mesure 16 peut avoir la forme d'un boîtier de mesure 16, tel qu'illustré sur la figure 7A. Selon un mode de réalisation, le module de mesure 16 et le module d'excitation 18 peuvent être intégrés à la partie souple 111 du bracelet 11.

Selon un autre mode de réalisation de l'invention, on peut envisager l'intégration du système 12 de traitement et d'affichage d'informations au sein du module de mesure 16.

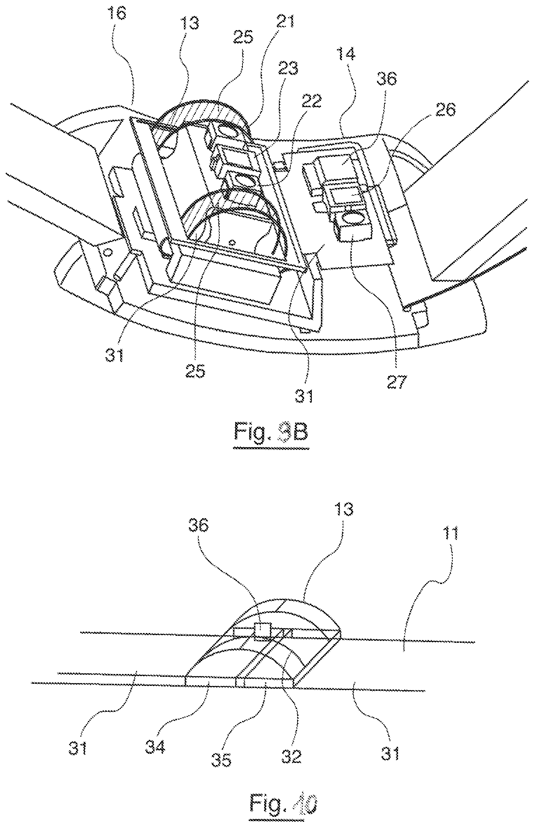

On présente, en relation avec les figures 9A et 9B, un schéma du module de mesure 16, selon un mode de réalisation de l'invention.

Il est à noter que le module d'excitation 18 n'a pas été représenté sur les figures 9A et 9B.

Comme décrit précédemment, le module de mesure 16 comprend un premier ensemble de mesure (qui est le palpeur 13 comprenant le premier ensemble de capteurs) et un second ensemble de mesure (constitué par le second ensemble de capteurs 14).

Ainsi, le palpeur 13 (ou premier ensemble de mesure) peut par exemple comprendre :
- une première diode électroluminescente 21 dont la longueur d'onde est, par exemple, voisine de 660 nm :
- une seconde diode électroluminescente 22 dont la longueur d'onde est, par exemple, voisine de 940 nm ;
- une photodiode 23 (encore appelée photodétecteur).

Les diodes électroluminescentes 21 et 22 et la photodiode 23 constituent des premiers capteurs optiques qui permettent notamment de réaliser des mesures pléthysmographiques à deux longueurs d'onde pour calculer le flot sanguin du sujet ou pour réaliser de l'oxymétrie afin de mesurer le taux d'oxygène dans le sang.

Le palpeur 13 (ou premier ensemble de mesure) peut en outre comprendre :
- deux électrodes 25 de mesure de résistance électrodermale (ou électrodes EDR pour « ElectroDermal Response » en anglais), qui sont des électrodes sèches par exemple espacées de 4 mm, chacune longue de 10 mm et large de 4 mm, et qui peuvent présenter des contacts surfaciques dorés ;
- un capteur (ou sonde) de température 36 par exemple associé à un amplificateur électrique qui permet d'obtenir la température cutanée de l'utilisateur. Le capteur de température peut par exemple être de type LM35 commercialisé par la société NATIONAL SEMICONDUCTOR (marque déposée). On considère dans la suite que l'amplificateur est compris dans le capteur de température. Bien entendu, l'amplificateur peut être externe au capteur de température. On peut ainsi obtenir la température cutanée à l'intérieur du poignet du sujet vivant avec une sensibilité de 110 mV/°C.

Le palpeur 13 peut également comprendre un capteur d'onde de pression (sensible à la variation de la tension artérielle du sujet) réalisé au moyen de cellules piézoélectriques 34 et 35 situées sur la partie inférieure plane du palpeur 13. Les deux cellules piézoélectriques sont, par exemple, du type PXE52 commercialisé par la société PHILIPS (marque déposée), et dont les dimensions sont par exemple 10 mm pour la longueur, 4 mm pour la largeur et 0,5 mm pour l'épaisseur.

Bien entendu, dans des variantes de ce mode de réalisation préférentiel, on peut mettre en oeuvre dans le palpeur 13 des cellules piézo-électriques de caractéristiques et de dimensions différentes.

Les deux cellules piézo-électriques 34 et 35 (présentes à la fois sur la figure 9A et sur la figure 10) sont par exemple montées en opposition (tête-bêche) dans un plan tangent au bracelet pour que les signaux provoqués par les contraintes latérales s'annulent, permettant ainsi de minimiser les effets de « bougé ». Elles peuvent être posées sur un matériau conducteur rigide qui assure leur protection (par exemple un clinquant métallique en cuivre de 0,5 mm d'épaisseur) et fiabilise le montage.

Sous l'effet d'une onde de pression transmise par la peau du porteur utilisateur, le palpeur 13 (par exemple réalisé en matériau plastique rigide non déformable) transmet à son tour une contrainte de pression sur toute la surface des cellules piézo-électriques 34, 35. Sous l'effet piézoélectrique direct, une charge est alors générée aux bornes des cellules piézoélectriques 34 et 35. Le préamplificateur 52 transformateur d'impédance transforme ensuite cette charge en tension.

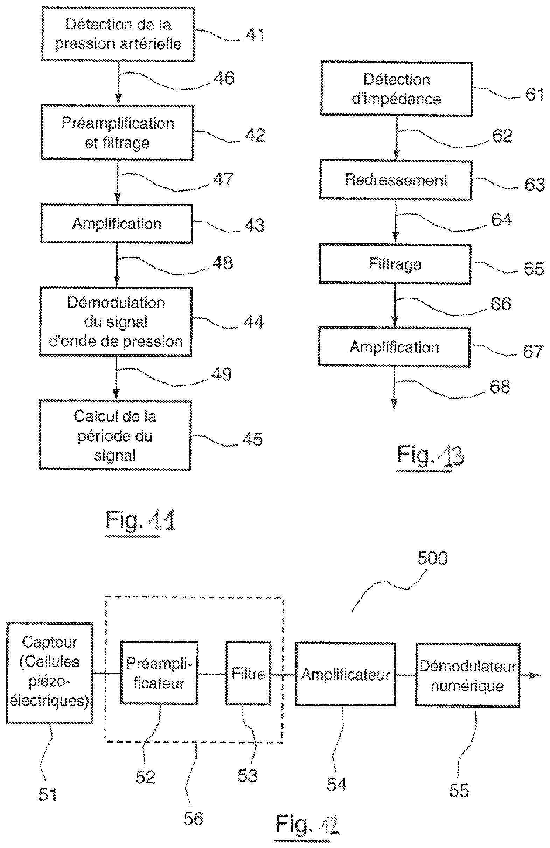

Par ailleurs, le premier ensemble de mesure peut comprendre des moyens de filtrage des signaux de mesure du premier ensemble de capteurs physiologiques comprenant un filtre 53 passe-bande (ci-après illustré sur la figure 12) réalisé notamment à partir d'une résistance et d'une capacité. Par exemple, la bande passante du filtre passe bande 53 est comprise entre 0,1 et 3 Hz.

Le second ensemble de mesure (constitué par le second ensemble de capteurs 14) sera décrit plus en détail ci-après.

### 6.3 Débruitage des signaux physiologiques mesurés

Comme décrit précédemment, le bracelet de mesure comprend un second ensemble de capteurs 14 (disposé sur le circuit imprimé 31), dédié au débruitage des signaux utiles mesurés par les capteurs du premier ensemble de mesure. Le second ensemble de capteurs 14 peut par exemple comprendre :
- une diode électroluminescente 27 dont la longueur d'onde est, par exemple, voisine de 660 nm ou de 940 nm. Bien entendu, selon une variante, le second ensemble de capteurs 14 peut comprendre plusieurs diodes électroluminescentes ;
- une photodiode 26.

Selon une variante, on peut envisager d'intégrer le capteur de température 36 au second ensemble de capteurs 14.

La diode électroluminescente 27 et la photodiode 26 constituent des seconds éléments optiques 26, 27 permettant d'isoler le bruit de mouvement.

Les seconds éléments optiques 26, 27 sont dédiés à la mesure de signaux de bruit quasiment pur destinés à être combinés aux signaux utiles (ou de mesure) issus des premiers capteurs optiques 21, 22, 23 afin de réduire l'influence du bruit sur ces signaux utiles. Le circuit imprimé 31 peut être un circuit souple conducteur (réalisé par exemple en kapton) de faible épaisseur (par exemple quelques dizaines de microns). Il peut être rapporté contre la partie souple 111 du bracelet 11, et s'étendre le long de la partie souple 111.

Les premier et second ensembles de mesure peuvent être disposés sur une des deux surfaces du circuit imprimé 31. Bien entendu selon une variante, d'autres composants (par exemple un amplificateur, des moyens de traitement numérique, ...) peuvent être soudés sur au moins une des deux surfaces du circuit imprimé 31.

Selon une autre variante, on peut envisager que le circuit imprimé 31 soit constitué de deux circuits imprimés distincts solidaires l'un de l'autre par exemple par un contact électrique. Selon encore une autre variante, le circuit imprimé 31 peut être réduit à un simple fil.

Par ailleurs, les cellules piézo-électriques peuvent être recouvertes par un matériau conducteur souple de faible épaisseur (par exemple quelques microns) jouant le rôle de contact électrique. Ainsi en recouvrant l'ensemble de la surface de chacune des cellules piézo-électriques, le matériau conducteur souple permet de maintenir la conduction électrique lorsqu'une des cellules piézo-électriques se casse.

Ce contact électrique constitue l'entrée d'un préamplificateur 52 transformateur d'impédance (ci-après illustré sur la figure 12).

Le palpeur 13 est par exemple situé droit au-dessus de l'artère radiale du poignet du sujet qui porte le dispositif de mesure et ainsi, chaque capteur du palpeur est situé au plus proche de l'artère qui est la source des signaux que l'on souhaite mesurer. En conséquence, chaque capteur du palpeur 13 peut délivrer un signal de mesure (autrement appelé signal utile) présentant un rapport signal sur bruit amélioré.

La face inférieure plane du palpeur 13 peut par exemple être maintenue plaquée sur la face supérieure de chacune des cellules piézo-électriques par des moyens élastiques (par exemple des ressorts). Selon une variante, la face inférieure plane du palpeur 13 peut être simplement collée sur la face supérieure des cellules piézo-électrique.

Une liaison glissière (non représentée) peut par exemple assurer le guidage vertical du palpeur par rapport à la surface supérieure des cellules piézo-électriques permettant de minimiser les frottements du palpeur 13 sur les contours d'un cadre 38 appartenant au boîtier extérieur 17 du module 16. Ainsi, la réception des contraintes mécaniques transmises à travers la peau du porteur au palpeur 13 (qui sont généralement de l'ordre de quelques dixièmes de Newton) est optimisée.

Selon une variante, un joint étanche peut être intégré au boîtier extérieur 17 du module de mesure pour réaliser l'étanchéité entre le palpeur 13 et les contours du cadre 38.

### 6.4 Electronique du bracelet de mesure

La figure 11 présente un organigramme des étapes principales d'un algorithme de traitement de l'onde mécanique de pression générée par l'artère radiale et utilisant le signal utile issu du capteur de pression (constitué par les cellules piézoélectriques 34 et 35) selon le mode de réalisation préférentiel de l'invention. L'algorithme de traitement est mis en oeuvre au sein d'un circuit électronique de traitement 500 de l'onde mécanique de pression intégré au bracelet de mesure et illustré sur la figure 12.

Tel qu'illustré par la figure 12, le circuit électronique de traitement 500 de l'onde mécanique de pression peut par exemple comprendre :
- un module capteur 51 comprenant les deux cellules piézoélectriques 34 et 35 ;
- un module 56 comportant le préamplificateur 52 monté en série avec le filtre passe bande 53 ;
- un amplificateur 54 ; et
- un démodulateur numérique 55 (intégré par exemple dans sous forme d'un algorithme dans un microcontrôleur).

Tel qu'illustré par la figure 11, dans une étape 41 de détection de l'onde de pression artérielle, les contraintes exercées sur les cellules piézo-électriques 34 et 35 par les ondes mécaniques de pression générées par l'artère radiale (la source) peuvent être sommées afin d'obtenir un signal de pression 46.

Dans une étape 42, le signal de pression 46 est par exemple préamplifié et filtré grâce au bloc électronique 56 comprenant le préamplificateur 52 ainsi que le filtre passe bande 53 montés en série. On obtient ainsi le signal de pression préamplifié et filtré 47.

Dans une étape 43, le signal de pression préamplifié et filtré peut être amplifié (on obtient ainsi un signal de pression amplifié 48 sur lequel se superpose une modulation d'amplitude AM et de fréquence FM) puis est ensuite démodulé numériquement (on obtient ainsi un signal de pression démodulé 49), dans une étape 44, de façon à extraire les paramètres respiratoires (la fréquence respiratoire et l'arythmie éventuelle) du signal de pression amplifié 48.

Dans une étape 45, la période du signal de pression démodulé 49 est calculée. Cette période correspondant à la période cardiaque du sujet et est équivalente à l'intervalle R-R.

On obtient alors la localisation exacte de chaque pic du pouls, ce qui permet de reconstituer l'intervalle R-R. Une procédure de débruitage est éventuellement appliquée préalablement si le niveau de bruit repéré est jugé trop conséquent.

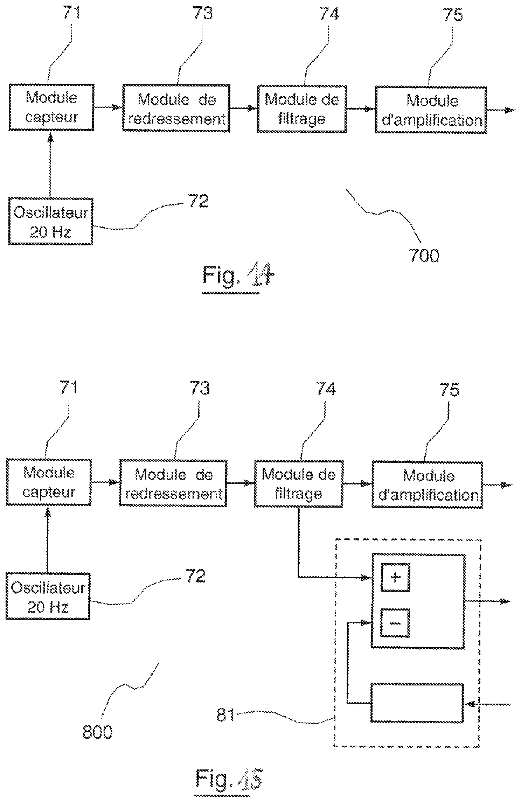

On présente, en relation avec la figure 13, un organigramme des étapes principales d'un algorithme de détermination de l'impédance de la peau du sujet utilisant le signal utile issu des électrodes EDR 25 selon un mode de réalisation de l'invention. L'algorithme de détermination est par exemple mis en oeuvre au sein d'un circuit électronique de détermination 700 de l'impédance de la peau intégré au bracelet de mesure et illustré sur la figure 14.

Tel qu'illustré par la figure 14, le circuit électronique 700 comprend par exemple:
- un module capteur 71 comprenant les deux électrodes EDR 25 ;
- un oscillateur électrique 72 oscillant à une fréquence d'oscillation de 20Hz environ ;
- un module de redressement 73 ;
- un module de filtrage 74 ; et
- un module d'amplification 75.

Tel qu'illustré par la figure 13, dans une étape 61, le module capteur 71 peut mesurer un signal électrodermal 62 (signal utile issu des électrodes EDR 25) qui est proportionnel à l'impédance de la peau du sujet. Pour ce faire, on peut par exemple mettre en oeuvre un signal alternatif à la fréquence d'oscillation précitée issu de l'oscillateur électrique 72 (le signal valant quelques µA) pour éviter l'effet de polarisation des électrodes.

Dans une étape 63, le signal électrodermal 62 est par exemple redressé par un module de redressement 73 (par exemple une diode et une capacité effectuant classiquement une détection de crête). On obtient ainsi un signal électrodermal redressé 64.

Dans une étape 65, le signal électrodermal redressé 64 peut être filtré par un module de filtrage 74 (qui est par exemple un filtre passe bande dont la bande passante s'étend entre 0,1Hz et 2Hz) afin d'obtenir un signal électrodermal filtré 66 qui est ensuite amplifié (par exemple d'un facteur 10) grâce aux moyens d'amplification 75 lors d'une étape 67. On obtient ainsi un signal électrodermal amplifié 68.

Selon une variante du mode de réalisation précité (des figures 13 et 14), illustrée par la figure 15, un circuit électronique 800 de détermination de l'impédance de peau peut comporter, outre les composants du circuit 700, une boucle de contre-réaction 81 supplémentaire permettant de supprimer la composante continue du signal électrodermal filtré 66.

Ainsi, deux signaux sont obtenus. La composante continue du signal est restaurée, permettant d'augmenter la dynamique (rapidité, précision, ...) du système, lors de la réponse à un stimulus ou de la surveillance simple de la résistance électrodermale par exemple.

Ainsi, le bracelet de mesure de l'invention, tel que décrit précédemment, minimise le bruit contenu dans les signaux de mesure en plaçant les capteurs physiologiques au plus près de l'artère radiale et en stabilisant le bracelet par rapport au poignet grâce à la protubérance ce qui peut permettre de gagner jusqu'à 20 dB par rapport aux mesures classiques.

Les seconds éléments optiques 26, 27 fournissent des signaux correspondant au bruit de mouvements du porteur (par exemple le bruit des tendons) et permettent de gagner jusqu'à 20 dB par rapport aux mesures classiques lorsqu'ils sont utilisés pour effectuer un débruitage optique.

### 6.5 Application à la détection de chute et/ou de malaise

Une application du dispositif décrit ci-dessus est envisagée pour la surveillance à distance d'un sujet vivant, par exemple un senior, en particulier pour la détection d'un malaise et/ou d'une chute.

Il est connu qu'un malaise et/ou une chute engendrent généralement un fort stress. On pourra donc utiliser le dispositif de mesure de l'état de stress décrit précédemment pour quantifier en continu ou périodiquement le niveau de stress de la personne âgée et émettre une alarme lorsque le niveau de stress mesuré, combiné éventuellement à d'autres mesures, permet de caractériser une situation problématique (dépassement d'un seuil prédéterminé). Cette alarme est par exemple transmise à distance vers des services d'aide d'urgence.

En d'autres termes, le malaise et/ou la chute peuvent constituer un stimulus « naturel » dans ce cas précis qui provoque une variation des paramètres physiologiques du porteur telle que la valeur de stress mesurée provoque le déclenchement d'une alarme.

De préférence, pour cette application, on mettra en oeuvre sur le bracelet un second ensemble de capteurs, tel que décrit précédemment, qui seront de natures différentes du premier ensemble de capteurs, de façon à effectuer des mesures basées sur des principes physiques différents. Le premier ensemble de capteurs peut ainsi être de nature mécanique et comprendre un capteur d'onde de pression réalisé au moyen de cellules piézoélectriques. Le second ensemble de capteurs peut être de nature optique et comprendre une ou plusieurs diodes électroluminescentes combinée(s) à une photodiode.

Les signaux du second ensemble de capteurs pourront être combinés, superposés ou comparés aux signaux issus du premier ensemble de capteurs. Par exemple, les signaux issus du second ensemble de capteurs seront dédiés au débruitage des signaux utiles mesurés par les capteurs du premier ensemble de mesure. Cette interprétation conjointe des signaux issus du premier et du second ensemble de capteurs permet de limiter le risque de fausses alarmes.

Le dispositif peut prendre en compte les signaux cardiaques et respiratoire, le signal de résistance de la peau et/ou la température locale de la peau par exemple. Chaque variable peut être pondérée et combinée aux autres variables par logique floue pour aboutir à un estimateur.

Dans un mode de réalisation de l'invention, le dispositif est en outre apte à détecter si le bracelet est porté ou non, par exemple sur la base des mesures de la température seule, d'une mesure optique seule ou de la résistance électrodermale seule, ou une combinaison de ces signaux.

### 6.6 Application à la détection de l'apnée du sommeil

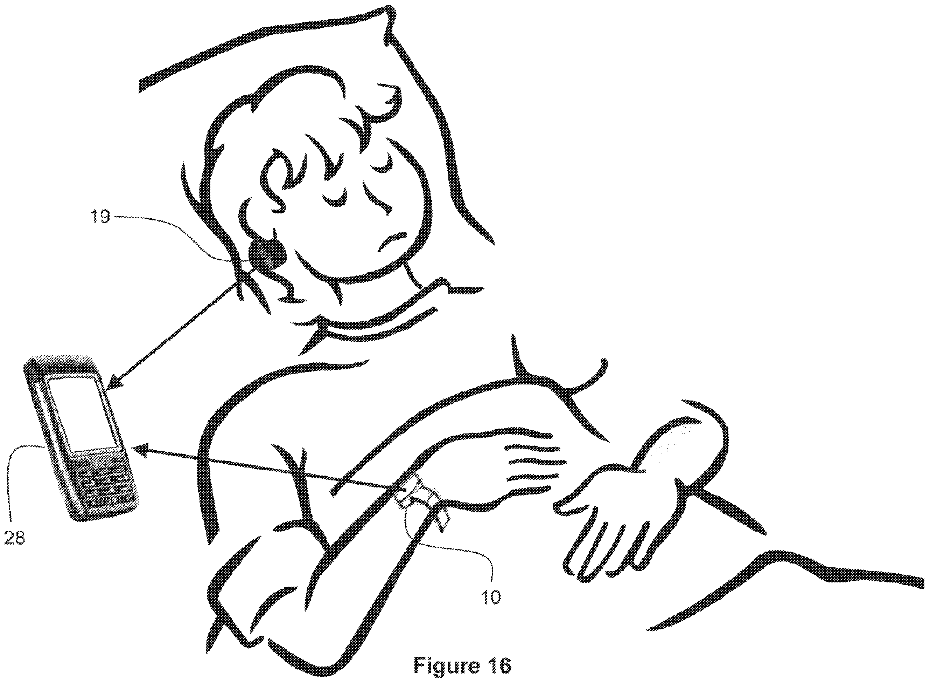

Une autre application du dispositif décrit ci-dessus est envisageable pour la détection de l'apnée du sommeil chez un sujet vivant. La figure 16 illustre un dispositif de détection de l'apnée du sommeil chez un sujet vivant mettant en oeuvre un dispositif de détermination d'un état de stress tel que décrit précédemment.

Dans cette application, le dispositif de mesure 10 de l'état de stress, porté ici au poignet par le sujet vivant, est mis en oeuvre avec un ou plusieurs capteurs sonores 19 supplémentaires de flux ou débit d'air et/ou de fréquence respiratoire par exemple, portés au niveau de chaque oreille par exemple. Le niveau de stress (ou l'ensemble ou une partie des données physiologiques), mesuré en continu ou périodiquement par le dispositif de mesure 10, et la mesure de flux d'air et/ou de fréquence respiratoire mesurée par le ou les capteurs 19 peuvent être, par exemple, transmis à distance vers un dispositif de télécommunications 28 (tel qu'un téléphone portable ou un terminal d'ordinateur), préférablement par l'intermédiaire d'une liaison sans fil.

Un tel dispositif de télécommunications 28 est notamment apte à mémoriser et traiter, par la mise en oeuvre d'une application logicielle, les données transmises par le dispositif de mesure 10 et le(s) capteur(s) 19, et à détecter une situation problématique.

En particulier, le dispositif de télécommunications 28 peut effectuer une analyse conjointe de la fréquence respiratoire mesurée par le ou les capteurs 19 et des paramètres respiratoires mesurés par le dispositif de mesure 10 pour déduire une situation d'apnée.

Une situation d'apnée peut par exemple correspondre à une chute de la fréquence cardiaque d'au moins 33% par rapport au rythme de base du sujet vivant.

Dans le cas où une apnée est diagnostiquée, une alarme de type sonore, visuelle ou autre peut être émise afin d'alerter l'utilisateur du dispositif de télécommunications 28 de cette situation, et une action peut alors être déclenchée.

On pourra envisager, dans une alternative ou en complément, que le dispositif de mesure 10 puisse communiquer par une liaison sans fil (de type Bluetooth® par exemple) avec le(s) capteur(s) 19 et effectuer une analyse conjointe de la fréquence respiratoire mesurée par le ou les capteurs 19 et des paramètres respiratoires mesurés par le dispositif de mesure 10, afin de détecter une situation d'apnée.

Dans le cas où une apnée est détectée, le dispositif de mesure 10 peut émettre un signal de stimulation sonore, visuelle, mécanique (vibreur tactile appliqué sur la peau du sujet vivant) et/ou olfactive, qui vise à réveiller le sujet vivant ou à le stimuler sans le réveiller pour qu'il reprenne sa respiration et son rythme cardiaque.

Un tel signal de stimulation pourra être émis par le module d'excitation 18 qui, tel que décrit précédemment, comprend une source électrique de stimulation à décharge piézo-électrique et est situé à proximité du palpeur 13.

Dans une variante, le signal de stimulation pourra être émis par le(s) capteur(s) 19 ou d'autres moyens non invasifs portés par le sujet vivant.

Le signal de stimulation peut prendre une valeur commandant une stimulation suffisante pour réveiller le sujet vivant, dans le cas d'apnées dangereuses ou répétées, et une valeur commandant une stimulation suffisamment faible pour ne pas réveiller le sujet vivant (on parlera de stimulation subliminale) dans le cas d'apnées non dangereuses. La stimulation peut également varier en fonction de différents paramètres (réglages par l'individu ou un praticien, évolution au cours du temps en fonction des précédentes stimulations, conditions externes (par exemple température ambiante, hygrométrie, ...), ...).

De manière préférentielle, le signal de stimulation varie en fonction de la réponse observée à la stimulation appliquée. Plus particulièrement, l'amplitude et/ou la fréquence du signal de stimulation est adaptée en fonction par exemple de la durée, de la fréquence et/ou de l'amplitude de l'apnée.

Pour la génération du signal de stimulation, on peut tenir compte par exemple d'une évolution du ou des signaux représentatifs de la fréquence cardiaque sur une période de temps prédéterminée..

Le signal de stimulation pourra prendre la forme d'un signal comprenant plusieurs impulsions d'intensités progressives jusqu'au réveil du sujet vivant, ou à tout le moins jusqu'à la fin de la situation d'apnée.

La fin d'une situation d'apnée étant de manière connue concomitante à un stress chez le sujet vivant (conduisant entre autres à une variation de la résistance électrodermale), celle-ci sera par exemple détectée par le dispositif de mesure 10 et/ou le dispositif de télécommunications 28 par analyse conjointe de la fréquence respiratoire mesurée par le ou les capteurs 19, des paramètres respiratoires et de l'impédance de la peau mesurés par le dispositif de mesure 10. L'utilisation de la mesure de la résistance électrodermale, et éventuellement d'autres paramètres physiologiques ou même d'un état de stress, permet ainsi de fiabiliser la détection d'une sortie d'apnée.

Pour un patient donné, le dispositif de mesure 10 pourra caractériser une apnée en mémorisant les paramètres physiologiques mesurés lors d'une première apnée afin d'obtenir une « signature ». Le dispositif de mesure 10 pourra alors détecter les apnées suivantes en comparant les paramètres physiologiques mesurés à cette signature mémorisée.

Ainsi donc, le dispositif de mesure 10 est donc adapté pour gérer une problématique d'urgence, par exemple lors d'une apnée du sommeil pouvant survenir de manière intempestive chez un sujet vivant. La mise en oeuvre du dispositif de mesure 10 proposée en relation avec la figure 16 répond au besoin croissant de réaliser une (télé)surveillance continue des données physiologiques des sujets sains ou pathologiques et de provoquer une action adaptative optimale, en fonction de l'état de santé du patient, localement ou à distance.

### 6.7 Variantes

Ainsi, le bracelet de mesure 10 selon l'invention permet notamment :
- une prise de mesures au plus près de la source (par exemple l'artère radiale) ;
- la mise en oeuvre d'un palpeur 13 permettant d'obtenir une réduction de la part de bruit des signaux mesurés par les capteurs physiologiques du bracelet (notamment le bruit d'artefact) ;
- la connaissance du système porté à partir de l'analyse de l'énergie délivrée par les cellules piézo-électriques 34 et 35, par les électrodes électrodermales 25, par le capteur 36 de température, ou par le retour des signaux optiques issus des premiers capteurs optiques ;

- la mesure de signaux issus des premiers capteurs optiques permettant le calcul de saturation en oxygène, validés par les signaux issus d'un capteur d'onde de pression ;
- la prise en compte de plusieurs paramètres physiologiques différents, permettant un traitement de données innovant ;
- la détermination d'une information représentative d'un état de stress.

Les signaux optiques et mécaniques présentent des caractéristiques corrélées, garanties notamment par l'architecture du palpeur 13. En cas de doute sur la qualité d'un type de signal, il est possible de vérifier la qualité de l'autre.

Par ailleurs, le bracelet de mesure selon l'invention peut comprendre des moyens de génération d'une alarme locale et des moyens de transmission vers un site distant de ladite alarme, après un délai prédéterminé, lorsque l'alarme n'a pas été invalidée manuellement par le sujet.

Le bracelet de mesure selon l'invention peut également comprendre des moyens de déclenchement (par exemple manuels) émettant une alarme vers un site distant lorsque au moins un des capteurs constate une anomalie appartenant au groupe suivant :
- absence d'énergie au niveau des capteurs d'ondes de pression piézo-électriques ;
- impédance supérieure à un seuil prédéterminé (par exemple 1MégaΩ) des électrodes de mesure de la résistance électrodermale ;
- variation supérieure à un seuil prédéterminé (par exemple 1°C) de la température indiquée par le capteur de température ;
- absence de modulation de flux indiqués par au moins une photodiode.

Le bracelet de mesure selon l'invention peut comprendre des moyens d'affichage d'au moins une des informations appartenant au groupe comprenant :
- heure et date ;
- indice de stress ;
- informations physiologiques ;
- alertes ;
- messages personnalisés, à un instant donné ;
- informations de localisation.

Bien entendu, un bracelet de mesure selon l'invention peut effectuer des mesures d'autres paramètres physiologiques et comprendre pour ce faire d'autres capteurs physiologiques.

Ces paramètres physiologiques peuvent être utilisés pour des fonctions complémentaires à celles de la détection du stress, telles que la détection des phases du sommeil, la détection de situations à risque, le stockage de données sur des périodes relativement longues à des fins d'analyse,...

Par ailleurs, le dispositif de l'invention ne prend pas nécessairement la forme d'un bracelet. En effet, les mesures physiologiques peuvent être à l'aide de capteurs appliqués sur d'autres zones du corps, à l'aide d'une ceinture, d'un collier, ou encore de moyens adhésifs.

Il peut en outre comprendre des moyens d'échange de données, par câble ou via une liaison hertzienne (par exemple de type Bluetooth, WiFi, radiotéléphone,...) pour stocker des données vers un ordinateur ou un terminal local, ou un serveur distant, ou encore pour émettre une alarme vers un superviseur, ou plus généralement une personne à avertir. Inversement, le dispositif de l'invention peut recevoir des données ou des commandes, par exemple pour lancer à distance une détection de stress.

## Revendications

1. Dispositif de détermination d'un état de stress chez un sujet vivant, comprenant au moins un module de mesure (205) d'au moins un paramètre physiologique dudit sujet, des moyens de génération (203) d'au moins un stimulus destiné à provoquer une réponse physiologique transitoire chez ledit sujet et des moyens de traitement (204) du ou des paramètres physiologiques mesurés par lesdits moyens de mesure après une durée de temporisation prédéterminée après ledit stimulus, délivrant au moins une information représentative dudit état de stress, **caractérisé en ce que** ledit module de mesure est monté sur un bracelet (11), destiné à être porté à un poignet dudit sujet, et porte des moyens de traitement alimentés par au moins un capteur de mesure prévu pour venir en contact avec la peau dudit sujet, au niveau dudit poignet, et **en ce que** ce que lesdits moyens de génération sont aptes à délivrer un premier stimulus, puis, après un laps de temps, une séquence prédéterminée de signaux de sondage, ledit laps de temps étant variable, de façon pseudo-aléatoire et/ou en fonction d'un état de stress déterminé préalablement.

2. Dispositif selon la revendication 1, **caractérisé en ce qu'**il comprend des moyens de mémorisation d'une grandeur représentative d'au moins un paramètre physiologique mesuré avant ledit stimulus, et des moyens de comparaison des grandeurs représentatives d'au moins un paramètre physiologique mesuré avant ledit stimulus et après ledit stimulus.

3. Dispositif selon la revendication 1 ou 2, **caractérisé en ce que** ladite séquence est variable de façon pseudo-aléatoire ou est fonction d'un état de stress déterminé préalablement à au moins un desdits signaux de sondage de ladite séquence.

4. Dispositif selon l'une des revendications précédentes, **caractérisé en ce que** lesdits moyens de génération comprennent au moins un des moyens appartenant au groupe comprenant :
- des moyens de génération d'un stimulus et/ou d'une séquence électrique ;
- des moyens de génération d'un stimulus et/ou d'une séquence visible ;
- des moyens de génération d'un stimulus et/ou d'une séquence sonore ;
- des moyens de génération d'un stimulus et/ou d'une séquence mécanique.

5. Dispositif selon la revendication 4, **caractérisé en ce que** lesdits moyens de génération d'un stimulus et/ou d'une séquence sonore délivrent au moins un bruit impulsionnel et/ou au moins un fichier audio.

6. Dispositif selon l'une des revendications précédentes, **caractérisé en ce qu'**il comprend des moyens de débruitage des signaux de mesure d'au moins un paramètre physiologique.

7. Dispositif selon l'une des revendications précédentes, **caractérisé en ce que** lesdits moyens de traitement comprennent des moyens de détection d'une situation d'apnée du sommeil tenant compte d'au moins une information représentative de la variabilité du rythme cardiaque dudit sujet vivant mesurée par lesdits moyens de mesure et d'au moins une grandeur représentative d'un flux d'air issue de moyens d'acquisition d'un signal représentatif d'un flux d'air.

8. Dispositif selon la revendication 7, **caractérisé en ce que** les moyens d'acquisition comprennent au moins un capteur sonore de flux d'air placé au voisinage de l'oreille dudit sujet vivant.

9. Dispositif selon la revendication 7 ou 8, **caractérisé en ce qu'**il comprend des moyens de communication avec lesdits moyens d'acquisition d'un flux d'air par l'intermédiaire d'une liaison sans fil.

10. Dispositif selon l'une des revendications 7 à 9, **caractérisé en ce qu'**il comprend des moyens de génération d'un signal de stimulation du sujet vivant mis en oeuvre dès lors qu'une situation d'apnée du sommeil est détectée.

11. Dispositif selon la revendication 10, **caractérisé en ce que** l'amplitude et/ou la fréquence du signal de stimulation est variable.

12. Dispositif selon la revendication 10 ou 11, **caractérisé en ce que** ledit signal de stimulation peut prendre au moins deux valeurs, dont au moins une valeur commandant une stimulation suffisante pour réveiller le sujet vivant et au moins une valeur commandant une stimulation ne réveillant pas le sujet vivant.

13. Dispositif selon l'une des revendications 10 à 12, **caractérisé en ce que** ledit signal de stimulation est un signal de stimulation électrique ou un signal de stimulation auditive.

14. Dispositif selon l'une des revendications 7 à 14, **caractérisé en ce qu'**il comprend des moyens de détermination de l'interruption d'une situation d'apnée du sommeil tenant compte d'au moins une information représentative de la variabilité du rythme cardiaque dudit sujet vivant, d'une grandeur représentative d'un flux d'air issue de moyens d'acquisition d'un flux d'air et d'une grandeur représentative de l'impédance de la peau.

15. Procédé d'aide à la détermination d'un état de stress chez un sujet vivant, mettant en oeuvre un dispositif selon l'une des revendications 1 à 14, **caractérisé en ce qu'**il comprend les étapes suivantes :
- génération d'au moins un stimulus destiné à provoquer une réponse physiologique transitoire chez ledit sujet ;
- mesure d'au moins un paramètre physiologique après une durée de temporisation prédéterminée après ledit stimulus, délivrant au moins une information représentative dudit état de stress ;
- analyse de ladite mesure ;
- délivrance d'au moins une information représentative dudit état de stress, en fonction de ladite analyse,
**caractérisé en ce qu'**il comprend en outre une étape de délivrance d'un premier stimulus puis, après un laps de temps, d'une séquence prédéterminée de signaux de sondage, ledit laps de temps étant variable de façon pseudo-aléatoire et/ou en fonction d'un état de stress déterminé préalablement.

16. Procédé selon la revendication 15, comprenant en outre les étapes suivantes :
- mesure d'au moins un paramètre physiologique avant ledit stimulus ;
- comparaison des mesures effectuées avant et après ledit stimulus.

17. Procédé selon la revendication 15 ou 16, **caractérisé en ce qu'**il comprend une étape de détermination d'une situation d'apnée du sommeil d'un sujet vivant tenant compte d'au moins une information représentative de la variabilité du rythme cardiaque dudit sujet vivant et d'au moins une grandeur représentative d'un flux d'air émis par ledit sujet vivant.

18. Procédé selon la revendication 17, **caractérisé en ce qu'**il comprend une étape de délivrance d'un signal de stimulation dudit sujet vivant dès lors qu'une situation d'apnée du sommeil est détectée.

19. Procédé selon la revendication 15 ou 17, **caractérisé en ce qu'**il comprend une étape de détection d'une chute et/ou d'un malaise et une étape de transmission d'une alarme à distance lorsque le stress mesuré dépasse un seuil prédéterminé.

20. Produit programme d'ordinateur téléchargeable depuis un réseau de communication et/ou stocké sur un support lisible par ordinateur et exécutable par un microprocesseur, permettant la mise en oeuvre, sur le dispositif selon l'une des revendications 1 à 14, du procédé de détermination de l'état de stress d'un sujet vivant selon l'une au moins des revendications 15 à 19, lorsqu'il est exécuté sur un ordinateur.

## Patentansprüche

1. Vorrichtung zur Bestimmung eines Stresszustands bei einem lebenden Subjekt, umfassend mindestens ein Modul (205) zur Messung mindestens eines physiologischen Parameters des Subjekts, Mittel (203) zur Erzeugung mindestens eines Stimulus, der dazu bestimmt ist, eine vorübergehende physiologische Reaktion bei dem Subjekt hervorzurufen, und Mittel (204) zur Verarbeitung des physiologischen Parameters oder der physiologischen Parameter, die durch die Messmittel nach einer zuvor bestimmten Verzögerungszeitdauer nach dem Stimulus gemessen werden, wobei mindestens eine Information geliefert wird, die für den Stresszustand repräsentativ ist,
**dadurch gekennzeichnet, dass** das Modul zur Messung an einem Armband (11) montiert ist, das dazu bestimmt ist, an einem Handgelenk des Subjekts getragen zu werden, und Mittel zur Verarbeitung trägt, die durch mindestens einen Messsensor beschickt werden, der dazu vorgesehen ist, mit der Haut des Subjekts an dem Handgelenk in Kontakt zu kommen,
und dass die Mittel zur Erzeugung geeignet sind, einen ersten Stimulus zu liefern, dann, nach einer Zeitspanne eine zuvor bestimmte Sequenz von Schallsignalen, wobei die Zeitspanne in pseudo-zufälliger Weise und/oder in Abhängigkeit von einem zuvor bestimmten Stresszustand variabel ist.

2. Vorrichtung nach Anspruch 1, **dadurch gekennzeichnet, dass** sie Mittel zur Speicherung einer Größe aufweist, die für mindestens einen physiologischen Parameter repräsentativ ist, der vor dem Stimulus gemessen wird, und Mittel zum Vergleich der repräsentativen Größen mindestens eines physiologischen Parameters, der vor dem Stimulus und nach dem Stimulus gemessen wird.

3. Vorrichtung nach Anspruch 1 oder 2, **dadurch gekennzeichnet, dass** die Sequenz in pseudo-zufälliger Weise variabel ist oder eine Funktion eines Stresszustandes ist, der zuvor bei mindestens einem der Schallsignale der Sequenz bestimmt wird.

4. Vorrichtung nach einem der vorhergehenden Ansprüche, **dadurch gekennzeichnet, dass** die Mittel zur Erzeugung mindestens eines der Mittel aufweisen, die zu der Gruppe gehören, die Folgendes aufweist:
- Mittel zur Erzeugung eines Stimulus und/oder einer elektrischen Sequenz;
- Mittel zur Erzeugung eines Stimulus und/oder einer sichtbaren Sequenz;
- Mittel zur Erzeugung eines Stimulus und/oder einer Schallsequenz;
- Mittel zur Erzeugung eines Stimulus und/oder einer mechanischen Sequenz.

5. Vorrichtung nach Anspruch 4, **dadurch gekennzeichnet, dass** die Mittel zur Erzeugung eines Stimulus und/oder einer Schallsequenz mindestens ein Impulsgeräusch und/oder mindestens eine Audiodatei liefern.

6. Vorrichtung nach einem der vorhergehenden Ansprüche, **dadurch gekennzeichnet, dass** sie Mittel zur Rauschunterdrückung der Messsignale mindestens eines physiologischen Parameters aufweisen.

7. Vorrichtung nach einem der vorhergehenden Ansprüche, **dadurch gekennzeichnet, dass** die Mittel zur Verarbeitung Mittel zur Detektierung einer Atemstillstandsituation im Schlaf unter Berücksichtigung mindestens einer Information, die für die Veränderlichkeit des Herzrhythmus des lebenden Subjekt repräsentativ ist, die durch die Messmittel gemessen wird, und mindestens einer Größe aufweisen, die für einen Luftstrom repräsentativ ist, der aus Mitteln zur Erlangung eines Signales stammt, das für den Luftstrom repräsentativ ist.

8. Vorrichtung nach Anspruch 7, **dadurch gekennzeichnet, dass** die Mittel zur Erlangung mindestens einen Luftstromschallsensor aufweisen, der in der Nähe des Ohres des lebenden Subjekts platziert ist.

9. Vorrichtung nach Anspruch 7 oder 8, **dadurch gekennzeichnet, dass** sie Mittel zur Kommunikation mit den Mitteln zur Erlangung eines Luftstromes durch eine drahtlose Verbindung aufweist.

10. Vorrichtung nach einem der Ansprüche 7 bis 9, **dadurch gekennzeichnet, dass** sie Mittel zur Erzeugung eines Signals zur Stimulierung des lebenden Subjekts aufweist, die eingesetzt werden, sobald eine Atemstillstandsituation im Schlaf detektiert wird.

11. Vorrichtung nach Anspruch 10, **dadurch gekennzeichnet, dass** die Amplitude und/oder die Frequenz des Signals zur Stimulierung variabel ist.

12. Vorrichtung nach Anspruch 10 oder 11, **dadurch gekennzeichnet, dass** das Signal zur Stimulierung mindestens zwei Werte annehmen kann, von denen mindestens ein Wert eine Stimulierung auslöst, die ausreicht, um das lebende Subjekt aufzuwecken, und mindestens ein Wert eine Stimulierung ausliest, welche das lebende Subjekt nicht aufweckt.

13. Vorrichtung nach einem der Ansprüche 10 bis 12, **dadurch gekennzeichnet, dass** das Signal zur Stimulierung ein Signal zur elektrischen Stimulierung oder ein Signal zur auditiven Stimulierung ist.

14. Vorrichtung nach einem der Ansprüche 7 bis 14, **dadurch gekennzeichnet, dass** sie Mittel zur Bestimmung der Unterbrechung einer Atemstillstandsituation im Schlaf unter Berücksichtigung mindestens einer Information, die für die Veränderlichkeit des Herzrhythmus des lebenden Subjekts repräsentativ ist, einer Größe, die für einen Luftstrom repräsentativ ist, der aus den Mitteln zur Erlangung eines Luftstromes stammt, und einer Größe aufweist, die für die Impedanz der Haut repräsentativ ist.

15. Verfahren zur Unterstützung bei der Bestimmung eines Stresszustandes bei einem lebenden Subjekt, bei dem eine Vorrichtung nach einem der Ansprüche 1 bis 14 eingesetzt wird, **dadurch gekennzeichnet, dass** es die folgenden Schritte aufweist:
- Erzeugung mindestens eines Stimulus, der dazu bestimmt ist, eine vorübergehende physiologische Reaktion bei dem Subjekt hervorzurufen;
- Messung mindestens eines physiologischen Parameters nach einer zuvor bestimmten Verzögerungszeitdauer nach dem Stimulus, wobei mindestens eine Information geliefert wird, die für den Stresszustand repräsentativ ist;
- Analyse der Messung;
- Lieferung mindestens einer Information, die für den Stresszustand repräsentativ ist, in Abhängigkeit von der Analyse,
**dadurch gekennzeichnet, dass** es außerdem einen Schritt zur Lieferung eines ersten Stimulus, dann, nach einer Zeitspanne, einer zuvor bestimmten Sequenz von Schallsignalen aufweist, wobei die Zeitspanne in pseudo-zufälliger Weise und/oder in Abhängigkeit von einem zuvor bestimmten Stresszustand variabel ist.

16. Verfahren nach Anspruch 15, außerdem die folgenden Schritte aufweisend:
- Messung mindestens eines physiologischen Parameters vor dem Stimulus;
- Vergleich der durchgeführten Messungen vor und nach dem Stimulus.

17. Verfahren nach Anspruch 15 oder 16, **dadurch gekennzeichnet, dass** es einen Schritt zur Bestimmung einer Atemstillstandsituation im Schlaf eines lebenden Subjekts unter Berücksichtigung mindestens einer Information, die für die Veränderlichkeit des Herzrhythmus des lebenden Subjekts repräsentativ ist, und mindestens einer Größe aufweist, die für einen Luftstrom repräsentativ ist, der von dem lebenden Subjekt ausgeht.

18. Verfahren nach Anspruch 17, **dadurch gekennzeichnet, dass** es einen Schritt der Lieferung eines Signals zur Stimulierung des lebenden Subjekts aufweist, sobald eine Atemstillstandsituation im Schlaf detektiert wird.

19. Verfahren nach Anspruch 15 oder 17, **dadurch gekennzeichnet, dass** es einen Schritt zur Detektierung eines Hinfallens und/oder eines Unwohlseins und einen Schritt der Übertragung eines Fernalarms aufweist, sobald der gemessene Stress eine zuvor bestimmte Schwelle übersteigt.

20. Computerprogrammprodukt, das von einem Kommunikationsnetz geladen werden und/oder auf einem Medium gespeichert werden kann, das von einem Computer lesbar und einem Mikroprozessor ausführbar ist, das die Umsetzung des Verfahrens zur Bestimmung des Stresszustandes eines lebenden Subjekts nach mindestens einem der Ansprüche 15 bis 19 auf der Vorrichtung nach einem der Ansprüche 1 bis 14 ermöglicht, wenn es auf einem Computer ausgeführt wird.

## Claims

1. Device for determining a state of stress of a living subject, comprising at least one measurement module (205) measuring at least one physiological parameter of the subject, generating means (203) generating a stimulus intended to provoke a physiological transient response of the subject and processing means (204) processing the physiological parameter(s) measured by the measurement module after a predetermined time lag following the stimulus, delivering at least one piece of information representative of the state of stress, **characterised in that** the measurement module is mounted on a bracelet (11) intended to be worn on the wrist of the subject, and carries processing means fed by at least one measurement sensor provided to come into contact with the skin of the subject, at the wrist, and **in that** the generation means are able to provide a first stimulus and then, following a time lag, a predetermined sequence of sampling signals, the time lag being variable, in a pseudo-random manner and/or as a function of a stress state determined in advance.

2. Device according to claim 1, **characterised in that** it comprises memory means for memorising a variable representative of at least one physiological parameter measured before the stimulus, and comparison means for comparing the representative variables of at least one physiological parameter measured before the stimulus and after the stimulus.

3. Device according to claim 1 or 2, **characterised in that** the sequence is variable in a pseudo-random manner or is a function of a state of stress determined previously at at least one of the sampling signals of the sequence.

4. Device according to any one of the preceding claims, **characterised in that** the generation means comprise at least one of the means belonging to the group comprising:
- means for generating a stimulus and/or an electrical sequence;
- means for generating a stimulus and/or a visible sequence;
- means for generating a stimulus and/or a sound sequence;
- means for generating a stimulus and/or a mechanical sequence.

5. Device according to claim 4, **characterised in that** the means for generating a stimulus and/or a sound sequence deliver at least one pulsed noise and/or at least one audio file.

6. Device according to any one of the preceding claims, **characterised in that** it comprises means for noise suppression of the measurement signals measuring at least one physiological parameter.

7. Device according to any one of the preceding claims, **characterised in that** the processing means comprise means for detecting a situation of sleep apnoea taking into account at least one piece of information representative of the variability of the heart rate of the living subject measured by the measurement means and at least one variable representative of an air flow provided by acquisition means for acquiring a signal representative of an air flow.

8. Device according to claim 7, **characterised in that** the acquisition means comprise at least one air flow acoustic sensor positioned in the vicinity of the ear of the living subject.

9. Device according to claim 7 or 8, **characterised in that** it comprises communication means for communicating with the air flow acquisition means through the intermediary of a wireless link.

10. Device according to any one of claims 7 to 9,
**characterised in that** it comprises generation means for generating a stimulation signal for stimulating the living subject activated when a situation of sleep apnoea is detected.

11. Device according to claim 10, **characterised in that** the amplitude and/or the frequency of the simulation signal is/are variable.

12. Device according to claim 10 or 11, **characterised in that** the stimulation signal can take at least two values, of which at least one value controls a stimulation that is sufficient to wake the living subject and at least one value controls a stimulation that does not wake the living subject.

13. Device according to any one of claims 10 to 12,
**characterised in that** the stimulation signal is an electrical stimulation signal or an audible stimulation signal.

14. Device according to any one of claims 7 to 14,
**characterised in that** it comprises means for determination of the interruption of a situation of sleep apnoea taking into account at least one piece of information representative of the variability of the heart rate of the living subject, a variable representative of an air flow provided by acquisition means for acquiring air flow and a variable representative of the skin impedance.

15. Method for assisting in determining the state of stress of a living subject, using a device according to any one of claims 1 to 14, **characterised in that** it comprises the following steps:
- generating at least one stimulus intended to provoke a physiological transient response of the subject;
- measuring at least one physiological parameter after a predetermined time lag following the stimulus, delivering at least one piece of information representative of the state of stress;
- analysis of the measurement;
- providing at least one piece of information representative of the state of stress as a function of the analysis,
**characterised in that** it also comprises a step of providing a first stimulus and then, following a time lag, a predetermined sequence of sampling signals, the time lag being variable in a pseudo-random manner and/or as a function of a state of stress determined in advance.

16. Method according to claim 15, also comprising the following steps:
- measuring at least one physiological parameter before the stimulus;
- comparing the measurements performed before and after the stimulus.

17. Method according to claim 15 or 16, **characterised in that** it comprises a step of determining a situation of sleep apnoea of a living subject, taking into account at least one piece of information representative of the variability of the heart rate of the living subject and at least one variable representative of an air flow issued by the living subject.

18. Method according to claim 17, **characterised in that** it comprises a step of providing a stimulation signal for stimulating the living subject when a situation of sleep apnoea is detected.

19. Method according to claim 15 or 17, **characterised in that** it comprises a step of detecting a fall and/or discomfort and a step of transmitting a remote alarm when the stress measured exceeds a predetermined threshold.

20. Computer program product downloadable from a communication network and/or stored on a computer-readable medium, executable by a microprocessor, allowing the implementation on the device according to any one of claims 1 to 14, of the method for determining the state of stress of a living subject according to at least one of claims 15 to 19, when executed on a computer.
